# EUROPEAN PATENT APPLICATION

(11) **EP 4 524 978 A1**
(43) Date of publication of application: **19.03.2025**
(21) Application number: 23803536.4
(22) Date of filing: 08.05.2023
(51) Int. Cl.: G16H 20/00, A61B 5/00, A61B 5/02, A61B 5/145, A61B 5/1455, G16Y 10/60, G16Y 20/40, G16Y 40/20

(54) **ANALYSIS DEVICE AND ANALYSIS METHOD**

(30) Priority: 09.05.2022 JP 2022077160; 09.05.2022 JP 2022077159
(71) Applicant: Kyocera Corporation, Kyoto-shi, Kyoto 612-8501 (JP)
(72) Inventor: NAGASAKA, Yushi, Kyoto-shi, Kyoto 612-8501 (JP); MORI, Hiroyuki, Kyoto-shi, Kyoto 612-8501 (JP); IKEDA, Yutaka, Kyoto-shi, Kyoto 612-8501 (JP); SHINTANI, Noriyuki, Kyoto-shi, Kyoto 612-8501 (JP); KAMEI, Kentaro, Kyoto-shi, Kyoto 612-8501 (JP)
(74) Representative: TBK
(86) International application number: PCT/JP2023/017288
(87) International publication number: WO 2023/219059

(57) **Abstract**

A meal time of a subject is estimated. An analyzer includes an acquisition unit that acquires a pulse wave of a subject continuously measured by a wearable device in a first period after a meal period in which the subject consumes a meal, a change detector that detects a pulse wave of the subject before a meal, and an index estimator that estimates a change in a metabolic index correlated with stiffness of a blood vessel of the subject.

## Description

### TECHNICAL FIELD

The present disclosure relates to an analyzer that analyzes pulse waves of a subject, and the like.

### BACKGROUND OF INVENTION

One of the objectives of dietary treatment which is one of the methods of treating diabetes is to curb rapid changes in blood glucose levels after having a meal. In order to continue such dietary treatment, it is important to make the subject recognize the connection between dietary composition and the responses of his or her body after consuming a meal.

Patent Document 1 discloses a technique of acquiring pulse waves of a subject and estimating a blood glucose level based on a metabolic index calculated from the waveform of the acquired pulse waves.

### CITATION LIST

### PATENT LITERATURE

Patent Document 1: Japanese Patent No. 6807481

### SUMMARY

An analyzer according to an aspect of the present disclosure includes an acquisition unit that acquires a pulse wave of a subject continuously measured by a wearable device in a first period after a meal period in which the subject consumes a meal, a detector that detects a pulse wave before the meal of the subject from pulse waves acquired by the acquisition unit, and an index estimator that estimates a change in a metabolic index correlated with stiffness of a blood vessel of the subject based on a change in the pulse wave detected by the detector.

An analysis method according to an aspect of the present disclosure includes an acquisition step of acquiring a pulse wave of a subject continuously measured by a wearable device in a first period after a meal period in which the subject consumes a meal, a detection step of detecting a pulse wave of the subject before the meal from acquired pulse waves, and an index estimation step of estimating a change in a metabolic index correlated with stiffness of a blood vessel of the subject based on a change in the detected pulse wave.

### BRIEF DESCRIPTION OF THE DRAWINGS

FIG. 1 is a diagram illustrating an example of an overview of an analysis system according to the present embodiment.
FIG. 2 is a diagram showing a state of a subject wearing a measuring device.
FIG. 3 is a functional block diagram illustrating a configuration example of an analysis system.
FIG. 4 shows graphs of examples of a change in a pulse wave detected by an analyzer.
FIG. 5 is a diagram illustrating a presentation example presented by the analyzer.
FIG. 6 is a diagram illustrating another presentation example presented by the analyzer.
FIG. 7 is a flowchart showing an example of a flow of processing by the analyzer.
FIG. 8 is a functional block diagram illustrating a configuration example of an analysis system according to another embodiment.
FIG. 9 is a functional block diagram illustrating a configuration example of an analysis system according to yet another embodiment.
FIG. 10 is a functional block diagram illustrating a configuration example of an analysis system according to yet another embodiment.
FIG. 11 is a diagram illustrating an example of an overview of an analysis system including a server.
FIG. 12 is a functional block diagram illustrating another configuration example of an analysis system.
FIG. 13 is a functional block diagram illustrating a configuration example of an analysis system.
FIG. 14 is a diagram illustrating a presentation example presented by the analyzer.
FIG. 15 is a diagram illustrating another presentation example presented by the analyzer.
FIG. 16 is a flowchart showing an example of a flow of processing by the analyzer.
FIG. 17 is a functional block diagram illustrating a configuration example of an analysis system according to another embodiment.
FIG. 18 is a functional block diagram illustrating a configuration example of an analysis system according to yet another embodiment.
FIG. 19 is a diagram illustrating an example of an overview of an analysis system including a server.
FIG. 20 is a functional block diagram illustrating another configuration example of the analysis system.

### DESCRIPTION OF EMBODIMENTS

It is not easy to make subjects recognize problems in their own dietary composition and establish proper dietary habits. In order to make a subject establish proper dietary habits, it is effective to take action in accordance with the time that has elapsed from the meal period in which the subject consumed a meal or the like.

An analyzer according to an aspect of the present disclosure provides an analysis system or the like in which a meal time that is an arbitrary time during a meal period of a subject can be estimated.

According to the analyzer according to an aspect of the present disclosure, a meal time of a subject can be estimated without receiving an active input from the subject. The analyzer can present the estimated meal time to the subject to prompt the subject to be aware of his/her meal time, and can take action on the subject according to the time that has elapsed from the meal time. The analyzer can enable the subject to recognize a problem (for example, fluctuating meal times) of his/her own dietary composition and thereby lead the subject to establish proper dietary habits.

In order to have a subject establish proper dietary habits, it is effective to enable the subject to recognize his or her dietary composition in association with the reaction of the subject's body after consuming the meal.

An analyzer according to an aspect of the present disclosure presents a subject with a message corresponding to the magnitude of a change in a metabolic index of the subject after a meal. Since the metabolic index is an index correlated with the stiffness of blood vessels, the magnitude of a change in the metabolic index of the subject after a meal can be said to be the magnitude of influence of the meal on the subject. Therefore, the analyzer can enable the subject to recognize his or her dietary composition in association with the reaction of the subject's body after he or she consumed the meal by presenting the subject with a message corresponding to the magnitude of change in the metabolic index. As a result, the analyzer can enable the subject to recognize the problem of his/her dietary composition and establish proper dietary habits.

### First Embodiment

### Outline

Insulin is a peptide hormone that plays an important role in glucose metabolism. Diabetes is a metabolic disorder caused by insufficient insulin action. Subjects suffering from or susceptible to diabetes (i.e., those with insufficient insulin secretion) see a significant increase in blood glucose levels after meals. Here, a meal refers to general consumption of nutrients that affect the endocrine system involved in metabolism, and is general food and drink including consumption of beverages including alcoholic beverages. A meal is not limited to those at meal times such as breakfast, lunch, and dinner.

The inventors of the present application have focused on the fact that the "vascular state" changes due to reactions of the secretory system such as a change in blood glucose level and insulin secretion before and after a meal, and as a result, the waveform of pulse waves changes. The inventors of the present application have found that a blood glucose level of a subject can be estimated, as well as a blood sugar-related index (metabolic index) affected by insulin secretion ability, and the like based on pulse waves of a subject. The inventors of the present application have invented an analyzer 10 and an analysis method that enable their own invention to be put into practice.

The analyzer 10 acquires a pulse wave of a subject continuously measured in a first period after a meal period in which the subject consumes a meal, and detects a pulse wave before the meal of the subject from acquired pulse waves. Then, the analyzer 10 estimates a metabolic index correlated with the stiffness of the blood vessel of the subject based on the detected change in the pulse wave. The analyzer 10 estimates the meal time of the subject based on a change in the estimated metabolic index. A meal time means a period between a meal start time at which a subject starts a meal and a meal end time at which the subject ends the meal, that is, an arbitrary time in a meal period which is a period during which the subject is eating. Here, a pulse wave of the subject in the first period is acquired using a wearable device (measuring device 20).

Accordingly, the analyzer 10 can estimate the metabolic index correlated with the stiffness of the blood vessel caused by the meal of the subject who has consumed the meal and the meal time based on the metabolic index. The analyzer 10 is capable of taking action according to the estimated meal time. Taking action according to a meal time may be, for example, presentation of the meal time to the subject, notification according to a time that has elapsed from the meal time, or the like. Accordingly, the analyzer 10 can enable the subject to recognize the information related to the meal time of the subject and the time that has elapsed from the meal time, and prompt the subject to make an effort to continue or improve his or her dietary composition and dietary habits.

An embodiment of the present disclosure will be described below.

### Analysis System 1

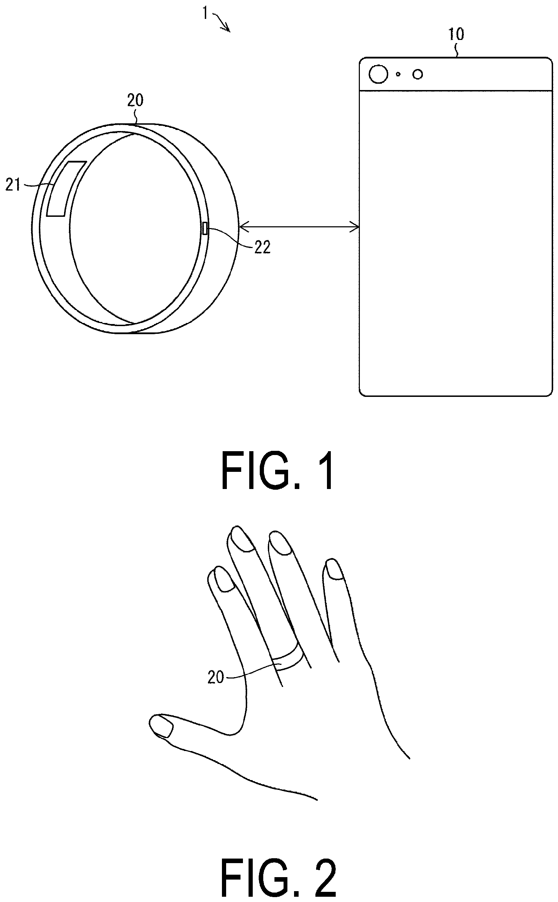

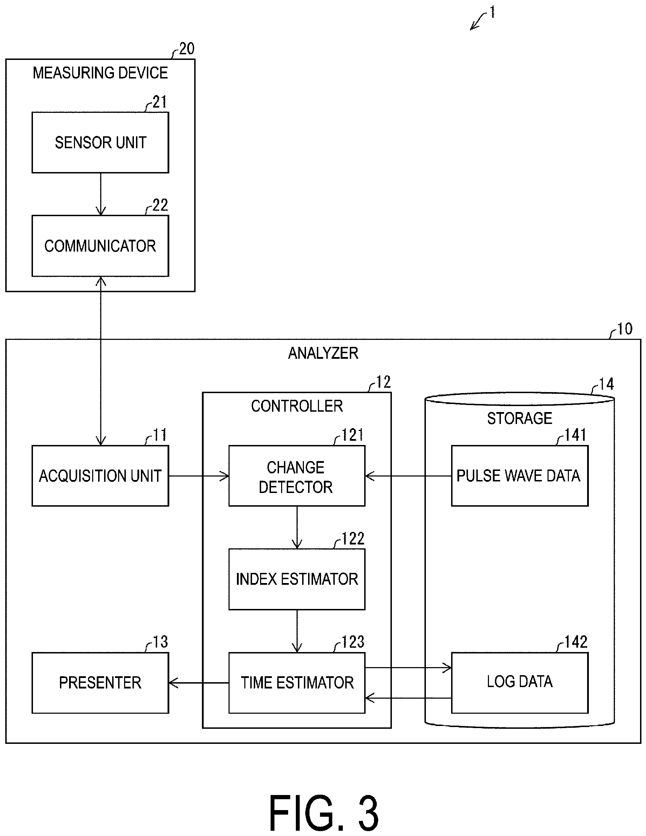

First, an overview of an analysis system 1 including the analyzer 10 according to the present embodiment will be described with reference to FIGs. 1, 2 and 3. FIG. 1 is a diagram illustrating an example of an overview of an analysis system 1. FIG. 2 is a diagram illustrating a state of a subject wearing a measuring device 20. FIG. 3 is a functional block diagram illustrating a configuration example of the analysis system 1. As illustrated in FIG. 1, the analysis system 1 includes the analyzer 10 and the measuring device 20. The analysis system 1 is a system in which the analyzer 10 analyzes a pulse wave of a subject measured by the measuring device 20 and presents the result to the subject. Details of the measuring device 20 and the analyzer 10 will be described below.

### Measuring Device 20

The measuring device 20 includes a sensor unit 21 and a communicator 22 as illustrated in FIG. 3.

The sensor unit 21 continuously detects displacement of a body surface of a subject and acquires a pulse wave. A pulse wave is obtained by capturing a temporal change in the volume of a blood vessel caused by inflow of blood as a waveform from a body surface. In the present specification, acquiring a pulse wave by the sensor unit 21 is referred to as measuring a pulse wave. Here, "continuously detect" means, for example, to detect displacement of a body surface of the subject at predetermined time intervals. The predetermined time intervals can be set to, for example, 1 minute, 10 minutes, 30 minutes, 1 hour, or the like. The predetermined time intervals may be changed according to a predetermined condition, for example.

The sensor unit 21 may detect the body surface of the subject at a higher measurement frequency in the first period than in other periods in accordance with an instruction from the analyzer 10. The measurement frequency may indicate, for example, the number of measurement operations in a predetermined period.

The communicator 22 wirelessly communicates with the analyzer 10 and transmits the pulse wave of the subject acquired by the sensor unit 21 to the analyzer 10 as pulse wave data.

The communicator 22 may transmit the pulse wave data to the analyzer 10 at a predetermined time when communication between the measuring device 20 and the analyzer 10 is established. The predetermined time may be a time designated by the analyzer 10.

For example, when communication between the measuring device 20 and the analyzer 10 is not established, the measuring device 20 stores the pulse wave acquired by the sensor unit 21 in a storage (not illustrated) as pulse wave data. When communication between the measuring device 20 and the analyzer 10 is established, the measuring device 20 transmits the pulse wave data stored until then to the analyzer 10, and transmits the pulse wave data to the analyzer 10 at a predetermined time while communication is established.

Rather than using the pulse wave data itself, the communicator 22 may perform data processing so that the analyzer 10 can easily analyze the pulse wave data and transmit processed pulse wave data after the data processing to the analyzer 10. When the volume of the processed pulse wave data is smaller than the volume of the pulse wave data, the volume of data to be transmitted to the analyzer 10 can be reduced.

The measuring device 20 is a wearable device that the subject can wear at all times. The measuring device 20 may have, for example, a shape of a ring that the subject can wear on a finger as illustrated in FIG. 1. FIG. 2 is a diagram illustrating a use example of the measuring device 20. FIG. 2 illustrates a state of a subject wearing the measuring device 20 on his or her right hand. Since the measuring device 20 is formed in a ring shape as illustrated in FIG. 2, the subject can always wear the measuring device 20 without feeling uncomfortable, and does not need to remove or put on the measuring device 20 for each time of measurement. By using the measuring device 20, the analysis system 1 can reduce the likelihood of measurement being forgotten or the like. Here, the measuring device 20 may be worn anywhere on the body of a subject as long as pulse waves of the subject can be continuously measured, and is not limited to being a wearable device worn on a finger. For example, the measuring device 20 may be a wristband type that enables the device to be worn around a wrist, or may have a collar shape that enables the device to be worn around a neck.

The measuring device 20 may include an acceleration sensor (not illustrated), a temperature sensor (not illustrated), and the like. If the measuring device 20 includes an acceleration sensor, the measuring device 20 can detect motions of a subject. If the measuring device 20 includes a temperature sensor, the measuring device 20 can measure the body temperature of a subject. For example, the analysis system 1 can determine a behavior (for example, walking, sleeping, eating, or the like) of the subject based on movement of the finger of the subject detected by the measuring device 20. The analysis system 1 can determine the health condition or the like of the subject based on the body temperature of the subject measured by the measuring device 20.

### Analyzer 10

The analyzer 10 will be described with reference to FIG. 3. As illustrated in FIG. 3, the analyzer 10 includes an acquisition unit 11, a controller 12, a presenter 13, and a storage 14. The controller 12 includes a change detector (detector) 121, an index estimator 122, and a time estimator 123.

The acquisition unit 11 acquires pulse wave data of the subject continuously measured by the measuring device 20 from the measuring device 20. The acquisition unit 11 may acquire pulse wave data of the subject continuously measured in a period (first period) after a meal period in which the subject consumes a meal. In the present specification, a "predetermined period after a subject actually has a meal" is referred to as a "first period". The first period may be, for example, a period from 30 minutes after a meal to one and a half hours after the meal, or a period from immediately after a meal to four hours after the meal. The analyzer 10 may continuously acquire pulse wave data during a period in which communication with the measuring device 20 is established. In this case, the pulse wave data acquired by the analyzer 10 may include the pulse wave data of the first period. As will be described below, after the subject finishes his or her meal, the pulse wave changes. The analyzer 10 analyzes the continuously measured pulse wave of the subject to detect a change in the pulse wave caused by the meal. By performing analysis based on the change, the analyzer 10 can estimate that the time at which the change has occurred is in the "first period". Thus, the analyzer 10 can estimate the meal time, which is the time at which the subject had the meal by acquiring and analyzing the pulse wave data of the period including the first period. As described above, the first period is a term for convenience that indicates a period during which change in the pulse wave can be detected, and the analyzer 10 does not need to ascertain the first period.

The acquisition unit 11 may acquire the pulse wave data at a predetermined frequency, and in this case, the acquisition frequency in the first period may be higher than the acquisition frequency in periods other than the first period. As a result, a change in a pulse wave after a meal can be quickly analyzed.

A pulse wave of the subject acquired by the acquisition unit 11 may include a pulse wave measured during a meal period of the subject. By including the pulse wave measured in the meal period of the subject, the pulse wave measured in the period can also be a target of analysis by the analyzer 10.

The pulse waves of the subject acquired by the acquisition unit 11 may include a period (second period) before the meal period of the subject. The pulse wave of the subject of a period before the meal period can be used by the change detector 121 (to be described below) to detect a change in the pulse wave of the subject.

The controller 12 controls the analyzer 10, and includes the change detector 121, the index estimator 122, and the time estimator 123 as described above.

The change detector 121 detects the pulse wave of the subject before a meal from pulse waves acquired by the acquisition unit 11 and detects a change from the pulse wave of the subject before the meal.

The change detector 121 may detect a change by comparing each beat of the pulse wave in the first period acquired by the acquisition unit 11 one beat at a time with that of the pulse wave before the meal of the subject. The change detector 121 may detect a change by comparing the pulse wave in a predetermined period (for example, 10 minutes) in the first period with the pulse wave before the meal of the subject in the same period as the predetermined period.

Here, when a metabolic index (for example, AI) to be described below is calculated, the metabolic index may be calculated by using a representative pulse wave shape generated by using the average value calculated by collecting a plurality of pulse waveforms of one beat in a predetermined period for "each beat of the pulse wave". Alternatively, for "each beat of the pulse wave", the metabolic index may be calculated from the pulse waveform of each of a plurality of beats in a predetermined period, and the average of the metabolic indexes may be used as the metabolic index in the predetermined period. More specifically, for example, when 70 pulse waves are acquired in one minute for "each beat of the pulse wave", the metabolic index may be calculated from each of the 70 pulse waves, and the average thereof may be set as the metabolic index for the one minute.

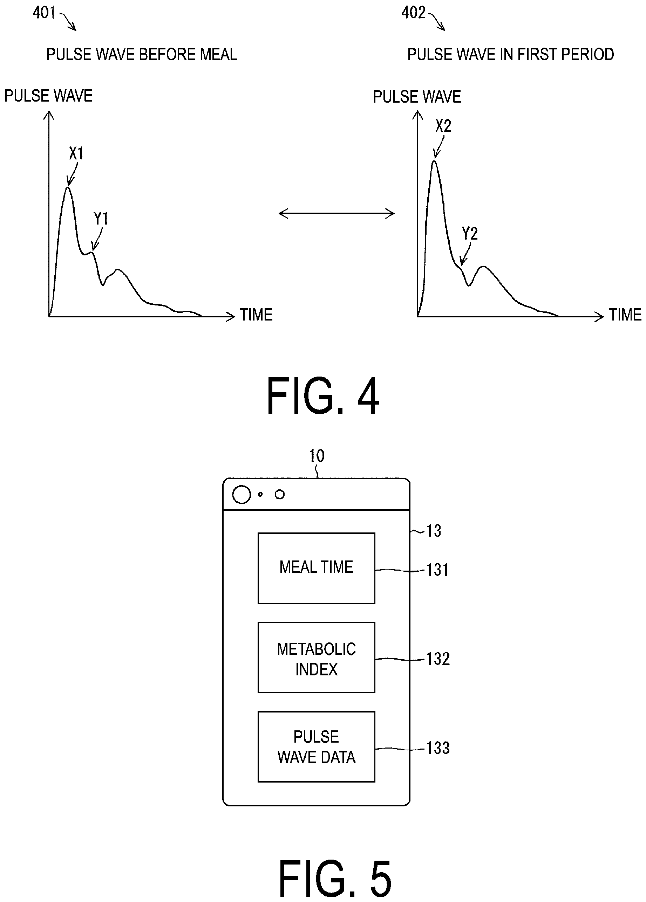

When a change is to be detected for each pulse wave corresponding to one beat, the change detector 121 may detect a change based on, for example, a difference in peak value, a difference in the falling manner, or the like. Here, a change detection process by the change detector 121 will be described with reference to FIG. 4. FIG. 4 shows graphs of examples of change in a pulse wave detected by the analyzer 10. A graph 401 in FIG. 4 is an example of the pulse wave before a meal, and a graph 402 is an example of the pulse wave in the first period. When the peak X1 of the graph 401 is compared with the peak X2 of the graph 402, it can be seen that the peak X2 of the graph 402 is greater than the peak X1 of the graph 401. With respect to the falling portions, it can be seen that the falling manner is different between the point Y1 of the graph 401 and the point Y2 of the graph 402. In this way, the change detector 121 detects a change in pulse wave from the difference between the peaks X1 and X2 and the difference between the points Y1 and Y2.

A change in a pulse wave may be detected from a ratio Y1/X1 of the second peak Y1 to the first peak X1 in the graph 401 and a ratio Y2/X2 of the second peak Y2 to the first peak X2 in the graph 402. That is, the change detector 121 may detect a change in a pulse wave depending on whether the ratio Y2/X2 with respect to the ratio Y1/X1 has changed by a predetermined value or more.

Referring back to FIG. 3, when detecting the change from the pulse wave in the predetermined period, the change detector 121 may detect the change by comparing the pulse wave in the predetermined period in the first period with the pulse wave in the predetermined period of the subject before a meal.

The index estimator 122 estimates a metabolic index correlated with the stiffness of the blood vessel of the subject based on the change detected by the change detector 121. Examples of the metabolic index include, but are not limited to, an augmentation index (AI) represented by a proportion in waveform magnitude of a forward wave of a pulse wave and a reflected wave region that appears later than the forward wave, and a pulse transit time (PTT) between a forward wave and a reflected wave of a pulse wave.

The index estimator 122 estimates the metabolic index based on, for example, an estimation formula created by using regression analysis. The estimation formula is stored in the analyzer 10 in advance.

Here, the estimation theory related to estimation of a metabolic index based on a pulse wave will be described. An increase in the blood glucose level in the blood after a meal causes a decrease in blood fluidity (an increase in viscosity), dilation of blood vessels, and an increase in the circulating blood volume, and angiokinetics and hemodynamics are determined to strike a balance in those states. The decrease in blood fluidity is caused by, for example, an increase in the viscosity of blood plasma or a decrease in the deformability of red blood cells. Dilation of blood vessels is caused by secretion of insulin, secretion of digestive hormones, increase in body temperature, and the like. When a blood vessel dilates, the pulse rate increases to curb the blood pressure from decreasing. An increase in a circulating blood volume also compensates for the amount of blood consumed for digestion and absorption. These factors also affect the stiffness of blood vessels.

Changes in angiokinetics and hemodynamics between before and after a meal caused by those factors are also reflected in pulse waves. Therefore, the index estimator 122 can estimate the metabolic index correlated with the stiffness of the blood vessels based on a change in the waveform of the pulse wave acquired via the acquisition unit 11.

Based on the above estimation theory, an estimation formula for estimating a metabolic index can be created by performing regression analysis based on sample data of pre-meal and post-meal metabolic indexes and pulse waves obtained from a plurality of subjects. At the time of estimation, the metabolic index of a subject can be estimated by applying the created estimation formula to the index based on the pulse wave of the subject. In the creation of the estimation formula, in particular, by creating the estimation formula by performing the regression analysis using the sample data in which the variation of the metabolic indexes is close to a normal distribution, the metabolic index of the subject to be examined can be estimated regardless of before or after the meal.

A metabolic index, for example, a value of an index AI (AI value), tends to decrease after a meal and reach the bottom peak some time after the meal. In other words, it is considered that a subject is having a meal at a time a certain amount of time before the metabolic index reaches the bottom peak. Therefore, by specifying the time at which the metabolic index reaches the bottom peak, the time estimator 123 can estimate the time dozens of minutes before the aforementioned time as the meal time of the subject.

The time estimator 123 acquires the metabolic index estimated by the index estimator 122 and estimates the meal time based on a change in the metabolic index. The time estimator 123 may specify a time point at which the metabolic index greatly changed, and estimate the meal time from the specified time point. For example, the time estimator 123 may specify a time at which the metabolic index is set in advance and rises from a value (reference value) serving as the reference of the change in the metabolic index and then reaches the bottom peak, and estimate the time dozens of minutes before (for example, 90 minutes before) the time as the meal time. The time estimator 123 may cause the presenter 13 to present the estimated meal time. The time estimator 123 may present the subject with other information according to the time that elapsed from the estimated meal time to the time at which the estimation is performed, or the like. The time estimator 123 may cause the storage 14 to store log data 142 including the estimated meal time. In the log data 142, the estimated meal time may be associated with other data such as a record of the corresponding metabolic index, a record of the pulse wave, and information indicating the time at which the estimation was performed.

In addition to the change in the metabolic index, the time estimator 123 may estimate the meal time of the subject based on the relationship between the past meal time of the subject and the information indicating the state of the subject at the past meal time. In this case, in the log data 142, a plurality of pieces of correct answer data related to the past meal times of the subject and pulse waves of the subject measured from each past meal time are stored in association with each other. The correct answer data is information indicating the time at which the subject actually had a meal. The correct answer data may be input by the subject himself or herself or may be input by a person who manages meals of the subject. The correct answer data may be recorded in association with the pulse waves of the subject at the meal time. The time estimator 123 may adjust an estimation parameter for estimating the meal time based on the correspondence relationship between the recorded plurality of pieces of correct answer data and the pulse waves associated with each piece of correct answer data. The time estimator 123 can improve the estimation accuracy of the meal time of the subject by using the adjusted estimation parameter for estimating the meal time based on the detected change in the metabolic index.

Other information may be associated with the correct answer data stored in the log data 142. For example, the meal time of the subject as the correct answer data may be associated with information indicating vibration caused by motions of the subject detected at the estimated meal time and information indicating the body temperature of the subject. In this case, the time estimator 123 may adjust the estimation parameter by further using these pieces of information. This makes it possible to further improve the accuracy of the meal time estimated by the time estimator 123.

A time at which the correct answer data is input is not particularly limited. As an example, the correct answer data may be input by the subject operating the measuring device 20 at a time when the subject has a meal. In this case, the measurement values of the pulse waves may be recorded more frequently during a predetermined period after the correct answer data is input. The correct answer data may be input at any time after the subject has the meal. In this case, the controller 12 records the input correct answer data in association with the information indicating the pulse waves of the subject at the time input as the correct answer data, which is already recorded in the log data 142.

As the reference value of the change in the metabolic index, a value gained during a period in which the metabolic index is stable and low may be used. For example, the time estimator 123 may specify the meal time based on the change in the metabolic index with respect to the criterion (first criterion), which is the metabolic index estimated based on the pulse waves of the subject at the time of wake-up or fasting. Here, the "time of wake-up" of the subject may refer to, for example, a time when the subject wakes up from sleep or may be defined by a sleep stage in sleep (for example, REM sleep close to wakefulness, etc.). The "time of fasting" of the subject may be, for example, a case in which the subject has not had a meal for a predetermined period (for example, a case in which a fasting period continues for 10 hours or longer) or a case in which the subject has taken an amount of food equal to or less than a predetermined reference amount.

A method for specifying the time of wake-up or the time of fasting of the subject is not particularly limited. As an example, the measuring device 20 may include an input unit (not illustrated) capable of inputting the time of wake-up or the time of fasting. In addition, as another example, the measuring device 20 may estimate the times of falling asleep and wake-up of the subject from the pulse of the subject, the acceleration indicating a change in posture, body surface temperature, and the like. The time of fasting may be automatically set after a predetermined time elapses from the intake of the meal, or the time of fasting may be estimated from a change in the pulse wave.

Alternatively, the time estimator 123 may use, as a criterion (first criterion), a metabolic index estimated based on a pulse wave of the subject measured in the past. The criterion may be, for example, the average of values of the metabolic indexes estimated in the past during a period in which variation is relatively small, or may be the lowest value of the metabolic indexes estimated in the past.

The time estimator 123 may refer to the log data 142 of the storage 14 to specify the criterion, and then specify the meal time of the immediately preceding meal of the subject based on the most recent change in the metabolic index with respect to the criterion. By using the metabolic index based on the pulse wave measured in the past as a criterion, how far the metabolic index fluctuates over time with respect to the past metabolic index can be specified, and the progress state of a chronic disease such as diabetes can be managed.

The presenter 13 presents the meal time estimated by the time estimator 123 to the subject. Examples of the presenter 13 include a display, a monitor, and the like. FIG. 5 is a diagram illustrating a presentation example presented by the analyzer 10. In the presentation example illustrated in FIG. 5, a meal time is presented in an area 131 of the presenter 13, a metabolic index is presented in an area 132, and pulse wave data is presented in an area 133.

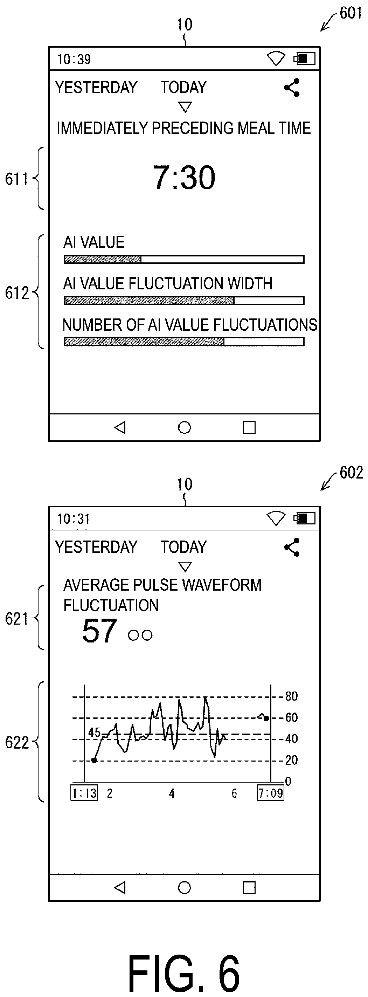

FIG. 6 is a diagram illustrating another presentation example presented by the analyzer 10. The analyzer 10 may give a presentation as illustrated in FIG. 6. Reference numeral 601 in FIG. 6 denotes another presentation example including a meal time. In reference numeral 601 of FIG. 6, a meal time is presented in an area 611, and information on an AI value as a metabolic index is presented in an area 612. Reference numeral 602 in FIG. 6 denotes a presentation example of a variation of a metabolic index. An AI value as a metabolic index is presented in an area 621 in reference numeral 602 in FIG. 6, and a varying state of the AI value is presented in an area 622.

The analyzer 10 may further present other information. When the difference between meal times during a predetermined period (for example, one week) exceeds a preset reference value, an evaluation result indicating that the meal times are irregular may be presented. As still another example, when the interval between a certain meal time and the next meal time of the subject is too large or too small, the analyzer 10 may present a message prompting the subject to have meals at proper time intervals.

The storage 14 stores data used by the analyzer 10, and includes pulse wave data 141 and log data 142.

The pulse wave data 141 is data of pulse waves of the subject before a meal, which is used by the change detector 121 to detect a change in a pulse wave. This may include pulse waves measured in advance or may include pulse waves measured before the immediately preceding meal. The log data 142 is data in which meal times estimated by the time estimator 123, and pulse waves, metabolic indexes, and the like used for the estimation are associated with each other.

As described above, the analyzer 10 according to the present disclosure includes the acquisition unit 11 that acquires a pulse wave of the subject continuously measured by using a wearable device (the measuring device 20) in the first period after the meal period in which the subject consumes a meal. The analyzer 10 also includes the change detector 121 (detector) that detects the pulse wave of the subject before the meal from pulse waves acquired by the acquisition unit 11. The analyzer 10 also includes the index estimator 122 that estimates a change in the metabolic index correlated with the stiffness of the blood vessel of the subject based on the change in the pulse wave detected by the change detector 121. The analyzer 10 includes the time estimator 123 that estimates a meal time, which is an arbitrary time during the meal period, based on the change in the metabolic index.

Thus, the analyzer 10 can estimate the meal time of the subject without receiving an active input from the subject. The analyzer 10 can present the estimated meal time to the subject to prompt the subject to be aware of his/her meal time, and can take action on the subject according to the time that has elapsed from the meal time. The analyzer 10 can enable the subject to recognize a problem (for example, fluctuating meal times) of his/her own dietary composition and thereby can enable the subject to establish proper dietary habits.

### Processing Flow

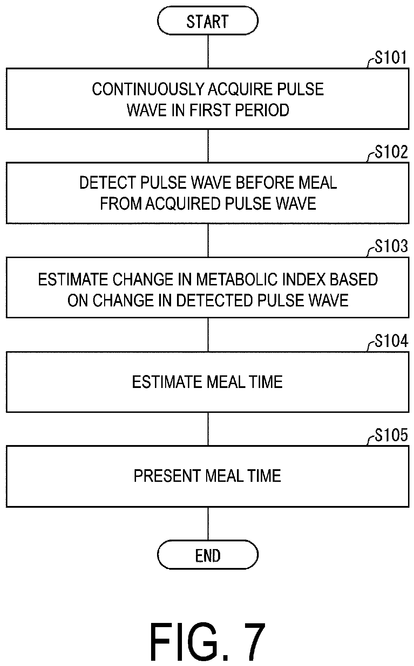

The flow of processing of the analyzer 10 will be described with reference to FIG. 7. FIG. 7 is a flowchart showing an example of the flow of processing by the analyzer 10. As shown in FIG. 7, the acquisition unit 11 acquires the pulse wave continuously measured by the measuring device 20 in the first period (S101; acquisition step). Next, the change detector 121 detects the pulse wave before the meal from pulse waves acquired by the acquisition unit 11 (S102; detection step). Then, the index estimator 122 estimates the metabolic index based on the change in the pulse wave detected by the change detector 121 (S103; index estimation step). Thereafter, the time estimator 123 estimates the meal time of the subject based on the change in the metabolic index estimated by the index estimator 122 (S104; time estimation step). Then, the presenter 13 presents the meal time of the subject estimated by the time estimator 123 (S105; presentation step).

The flow of processing by the analyzer 10 is as described above.

### Second Embodiment

Another embodiment of the present disclosure will be described below. For convenience of description, members having the same functions as those of the members described in the above-described embodiment are denoted by the same reference signs, and description thereof is not repeated. In the present embodiment, description of contents overlapping with those of the above-described embodiment will be omitted.

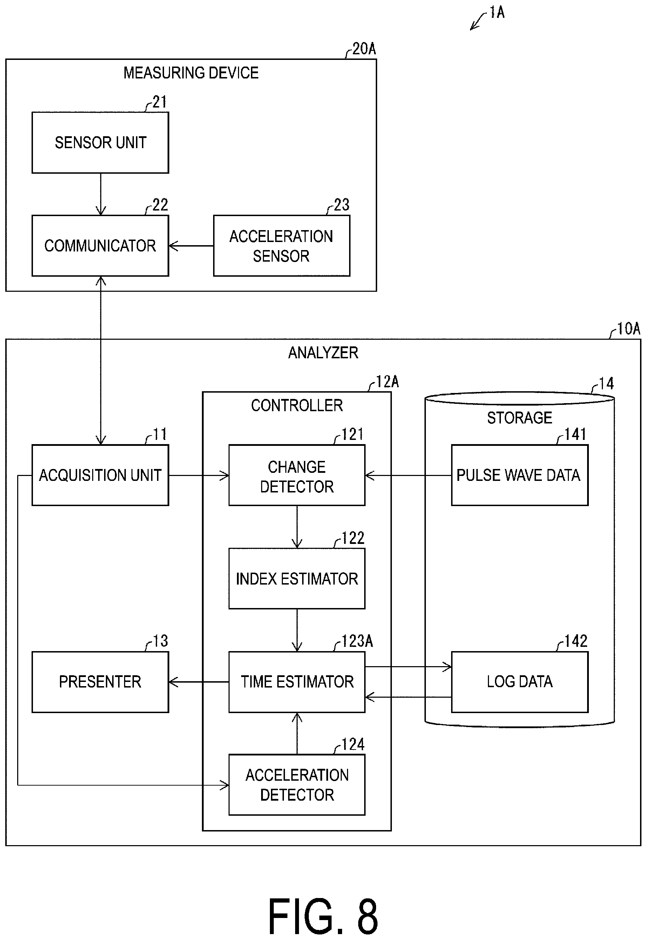

FIG. 8 is a principal configuration of an analysis system 1A according to the present embodiment. As illustrated in FIG. 8, the analysis system 1A includes a measuring device 20A and an analyzer 10A. As illustrated in FIG. 8, the measuring device 20A differs from the measuring device 20A according to the above-described first embodiment in that the former includes an acceleration sensor 23.

The analyzer 10A includes a controller 12A including a time estimator 123A as illustrated in FIG. 8. The analyzer 10A differs from the analyzer 10A according to the above-described first embodiment in that the former includes a controller 12A including a time estimator 123A and an acceleration detector 124 instead of the controller 12. In the analysis system 1A according to the present embodiment, the time estimator 123A of the analyzer 10A estimates a meal time of the subject based on a combination of a metabolic index and another index. As an example, the time estimator 123A estimates a meal time based on the metabolic index and the acceleration of a body motion of the subject.

The acceleration sensor 23 is a sensor capable of outputting an output value corresponding to an acceleration of a body motion of the subject. A body motion of the subject is a motion, for example, moving, standing, or sitting of the subject. Alternatively, the acceleration sensor 23 may be a sensor capable of outputting an output value corresponding to a motion performed by the subject during eating as a body motion of the subject, for example, a motion such as lifting or lowering of tableware or chewing.

The acceleration detector 124 acquires the output value corresponding to the acceleration of the body motion of the subject from the acceleration sensor 23 via the acquisition unit 11, and detects what kind of motion the subject performed. For example, the acceleration detector 124 detects that the subject has moved and then sat down based on the acquired output value. The acceleration detector 124 outputs information indicating the detected motion of the subject to the time estimator 123A.

The time estimator 123A acquires information indicating the subject's motion detected by the acceleration detector 124 in addition to information indicating the metabolic index estimated by the index estimator 122, and estimates the meal time of the subject based on these pieces of information. In other words, the time estimator 123A estimates the meal time of the subject based on the metabolic index and the acceleration of the body motion.

For example, when the metabolic index increases after the subject moves and sits down, the time estimator 123A estimates that the metabolic index has increased due to the subject sitting down and having a meal, and estimates the meal time based on the change in the metabolic index. As described above, the time estimator 123A can improve the estimation accuracy of the meal time of the subject by performing estimation using another index in addition to the metabolic index. A motion of the subject used by the time estimator 123A to estimate the meal time of the subject is not limited to moving and sitting, and may be any motion of the subject related to having a meal. A motion of the subject related to having a meal may be, for example, motions of the subject walking, then standing still, and then walking again. When such a motion is detected, the time estimator 123A may estimate the meal time by considering that there is a possibility that the subject had a meal at any time while the subject is standing still, and combining the information indicating the motion and the metabolic index of the subject.

The time estimator 123A may estimate the meal time in more detail. For example, the time estimator 123A may estimate at least one of a mealtime start time and a meal end time of the subject based on the metabolic index and the acceleration of the body motions. To be more specific, the time estimator 123A may estimate a time before the meal time estimated based on the metabolic index and at which the subject being seated is detected based on the output value of the acceleration sensor 23 as the mealtime start time of the subject. The time estimator 123A may estimate, as the meal end time of the subject, a time after the estimated meal time at which the subject is detected to be standing or a time at which an action of the subject involved with a meal by the subject is stopped. The time estimator 123A may determine whether to estimate the meal time based on the acceleration of the body motions of the subject. For example, when the metabolic index increases while the subject is moving, the time estimator 123A may determine that the increase in the metabolic index is not caused by a meal and may not estimate a meal time.

### Variation

In the analysis system 1A according to the present embodiment, the time estimator 123A of the analyzer 10A may use an index other than accelerations of the body motions of the subject as another index to be combined with the metabolic index. As an example, the time estimator 123A may estimate a meal time based on the metabolic index and the body surface temperature of a subject. In this case, the measuring device 20A includes a temperature sensor capable of measuring the body surface temperature of the subject, and transmits information indicating the body surface temperature of the subject to the analyzer 10A. The controller 12A of the analyzer 10A acquires information indicating the body surface temperature of the subject in addition to information indicating pulse waves of the subject. The time estimator 123A estimates the meal time of the subject based on the information indicating the body surface temperature of the subject in addition to the metabolic index estimated by the index estimator 122. In general, when a subject has a meal, the body surface temperature of a limb of the subject tends to increase after the meal. In other words, when a change in the metabolic index and an increase in the body surface temperature of the subject occur, there is a high likelihood that the subject had a meal at a time before the body surface temperature increases. Therefore, the time estimator 123A can more accurately estimate the meal time of the subject by ascertaining the time at which the body surface temperature of the subject rose and performing estimation in combination with the metabolic index. The time estimator 123A may perform estimation by combining the metabolic index of the subject and two or more other indexes. For example, the time estimator 123A may estimate the meal time by combining the metabolic index of the subject, the accelerations of body motions of the subject, and the body surface temperatures of the subject.

### Third Embodiment

Another embodiment of the present disclosure will be described below. For convenience of description, members having the same functions as those of the members described in the above-described embodiment are denoted by the same reference signs, and description thereof is not repeated. In the present embodiment, description of contents overlapping with those of the above-described embodiment will be omitted.

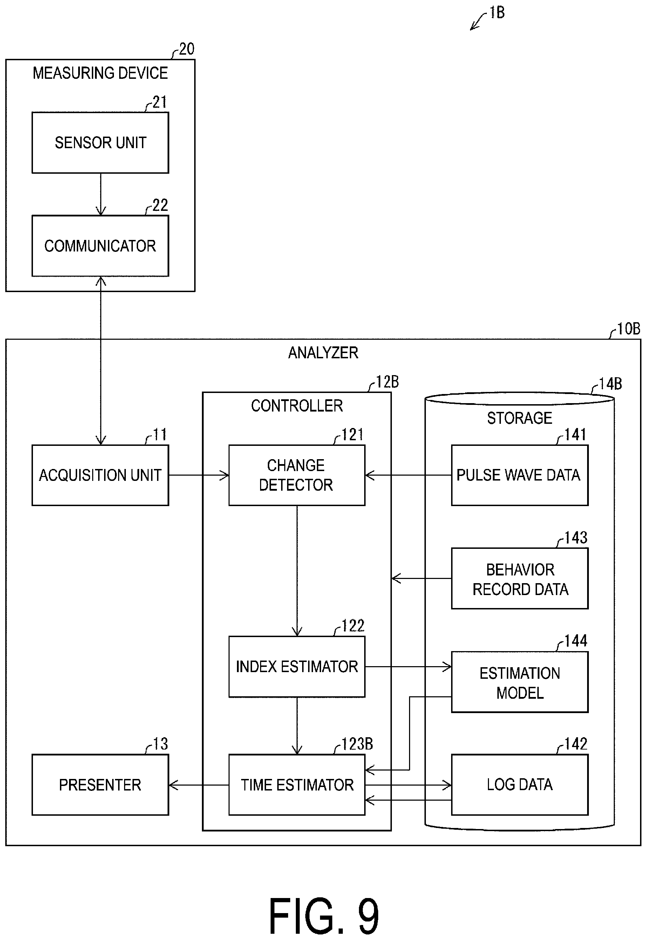

FIG. 9 is a principal configuration of an analysis system 1B according to the present embodiment. As illustrated in FIG. 9, the analysis system 1B includes an analyzer 10B and a measuring device 20. The analysis system 1B is different from the analysis system 1 according to the above-described first embodiment in that the former includes the analyzer 10B instead of the analyzer 10. As illustrated in FIG. 9, the analyzer 10B includes a controller 12B and a storage 14B. The analyzer 10B is different from the analyzer 10 according to the above-described first embodiment in that the former includes the controller 12B including a time estimator 123B instead of the controller 12 including the time estimator 123. The analyzer 10B is different from the analyzer 10 in that the former includes the storage 14B storing behavior record data 143 and an estimation model 144 in addition to the data stored in the storage 14.

The behavior record data 143 is data indicating behavior of a subject, such as a meal time, a meal start time, a meal end time, a meal period, and a period from the end of one meal to the start of the next meal of the subject. The behavior record data 143 of a subject may be data input to the analyzer 10B by the subject or another person, or may be data indicating behavior detected by a sensor capable of detecting behavior of the subject, such as a temperature sensor or an acceleration sensor, which are not illustrated. The analyzer 10B may acquire and use behavior record data of the subject from an external device that stores behavior record data of the subject. The estimation model 144 is a trained estimation model obtained by performing training based on each piece of data included in past behavior records of the subject, for example, the behavior record data 143 of the subject.

When a metabolic index is estimated, the time estimator 123B uses the estimation model 144 to specify behavior of the subject, for example, a meal time or the like of the subject based on change in the metabolic index. That is, the time estimator 123B estimates the meal time based on a combination of the metabolic index and the training result based on past behavior record of the subject.

The time estimator 123B may estimate at least one of the meal start time and the meal end time based on the combination of the metabolic index and the training result based on the past behavior record of the subject.

### Fourth Embodiment

Another embodiment of the present disclosure will be described below. For convenience of description, members having the same functions as those of the members described in the above-described embodiment are denoted by the same reference signs, and description thereof is not repeated. In the present embodiment, the description of contents overlapping with those of the above-described embodiment will be omitted.

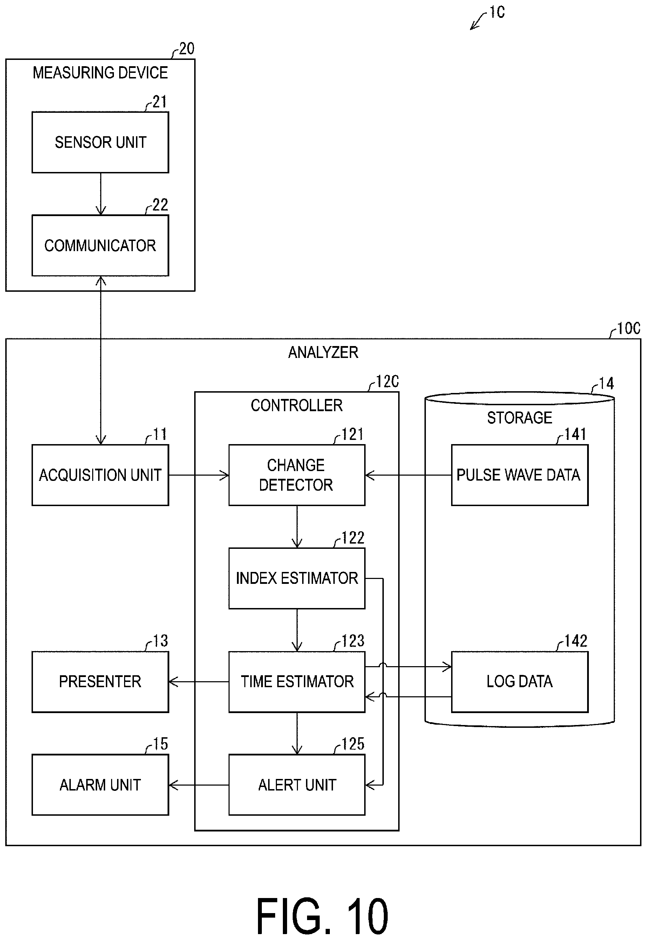

FIG. 10 is a principal configuration of an analysis system 1C according to the present embodiment. As illustrated in FIG. 10, the analysis system 1C includes an analyzer 10C and a measuring device 20. The analysis system 1C is different from the analysis system 1 according to the above-described first embodiment in that the former includes an analyzer 10C instead of the analyzer 10. As illustrated in FIG. 10, the analyzer 10C includes a controller 12C including an alert unit 125 and an alarm unit 15. The analyzer 10C is different from the analyzer 10 according to the above-described first embodiment in that the controller 12C includes the alert unit 125 and the alarm unit 15.

The alert unit 125 causes the alarm unit 15 to issue various alerts in accordance with preset conditions. The preset conditions may be, for example, a condition related to an estimated metabolic index or a meal time of a subject.

For example, when the metabolic index is low or is expected to be low, the alert unit 125 outputs an alert prompting the subject to consume food or the like. As an example, the alert unit 125 acquires an estimated metabolic index, and outputs a predetermined alert to the subject when the metabolic index falls below a preset second criterion.

For example, if the metabolic index indicates a blood glucose level, the second criterion may be a criterion by which the subject can be evaluated as being in a hypoglycemic state when the metabolic index falls below the criterion.

The content of the alert output by the alert unit 125 may be at least one of notification of the situation or presentation of a message for calling attention to the subject or the destination designated by the subject. More specifically, the alert may be, for example, a message indicating "The metabolic index has fallen below the reference value", "How about refreshing yourself by having a snack or water?", or "The metabolic index has fallen below the reference value. How about refreshing yourself by having a snack or water?". When the time from the estimated meal time exceeds a third criterion, in other words, when a certain amount of time has elapsed since the immediately previous meal, the alert unit 125 may output an alert prompting the subject to consume food. By outputting an alert when the metabolic index is low or when time has elapsed since the previous meal, the subject can made to recognize that consuming food or the like is desirable even if the subject himself/herself is not aware of the bad condition.

For example, when the metabolic index indicates a blood glucose level, the third criterion may be a criterion by which it can be estimated that the subject is in a low blood glucose state when the subject has not consumed a meal for a period of time exceeding the criterion.

When the estimated metabolic index falls below a preset fourth criterion, the alert unit 125 may output advice on the next meal. The next meal may mean lunch after breakfast on a certain day or may mean breakfast on the next day.

For example, when the metabolic index is a value related to the dietary composition, the fourth criterion may be a criterion by which the dietary composition of the subject adversely affects his or her health when the metabolic index falls below the criterion.

The content of the advice may be, for example, a message indicating "A possibility that exercise was performed after the meal has been detected. Avoid performing exercise immediately after a meal". The analysis system 1C may include an acceleration sensor (not illustrated) capable of detecting whether the subject is exercising, and may give advice in which the exercise of the subject is associated with the time of the meal. For example, when a meal time of the subject is detected and exercise of the subject is detected after the meal time, a message indicating "Exercise has been detected. You may feel hungry since you acted more than usual, so take your time to chew the food" may be presented to the subject as advice.

The alarm unit 15 issues an alert in accordance with an instruction from the alert unit 125. The alarm unit 15 may output the alert by sound, or may output the alert by emitting a color that attracts the eyes of the subject, such as red or yellow. In addition, the alarm unit 15 may output a message by text or voice as the alert. The alarm unit 15 may be formed integrally with the presenter 13. The alarm unit 15 may be provided in the measuring device 20. In this case, the alert unit 125 instructs the alarm unit 15 via the communicator 22 of the measuring device 20 to cause the alarm unit 15 to output an alert.

### First Variation

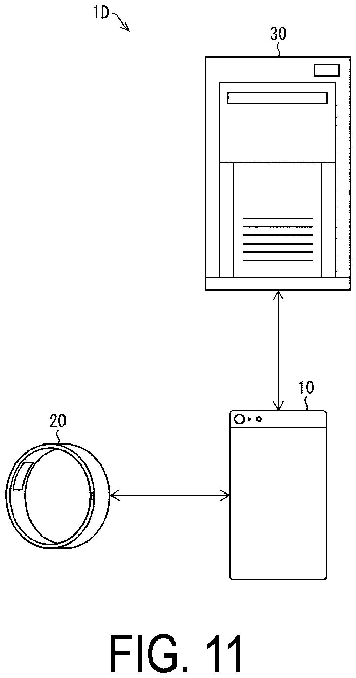

FIG. 11 is a diagram illustrating an example of an overview of an analysis system 1D including a server 30. As illustrated in FIG. 11, the analysis system 1D further includes the server 30 in addition to the analyzer 10 and the measuring device 20 described above.

The analyzer 10 and the server 30 are communicatively connected to a network.

The server 30 performs a part or all of the analysis processing performed by the analyzer 10. Since the server 30 performs the analysis processing, the analyzer 10 can reduce processing load.

### Second Variation

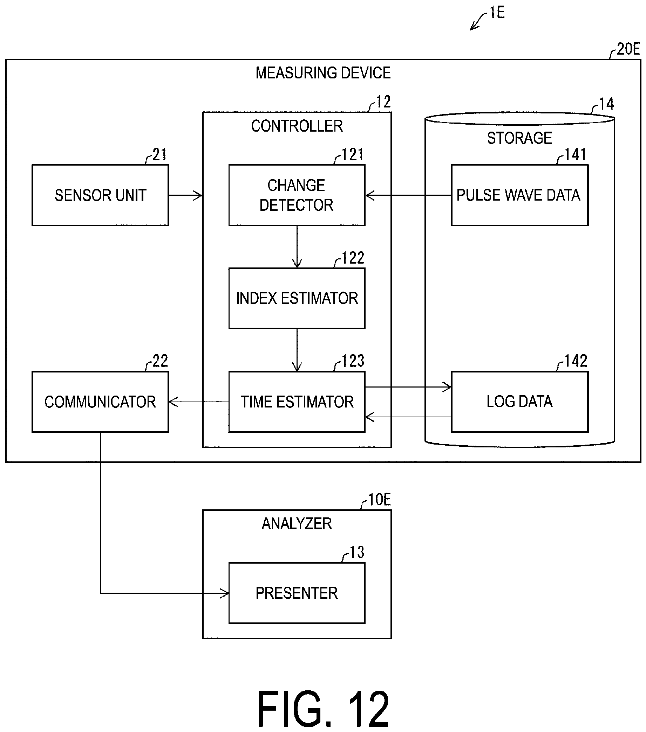

The analyzer 10 analyzes pulse waves measured by the measuring device 20 in the embodiments described above. However, the present disclosure is not limited thereto, and a part of the functions of the analyzer 10 may be assigned to the measuring device 20. An analysis system 1E employing such a configuration will be described with reference to FIG. 12. FIG. 12 is a functional block diagram illustrating a configuration example of the analysis system 1E. A measuring device 20E of the analysis system 1E measures and analyzes pulse waves, and an analyzer 10E acquires the analysis result acquired from the measuring device 20E and presents the analysis result on a presenter 13.

As illustrated in FIG. 12, the measuring device 20E includes a controller 12 (a change detector 121, an index estimator 122, and a time estimator 123) and a storage 14 (pulse wave data 141 and log data 142) in addition to a sensor unit 21 and a communicator 22. The measuring device 20E performs analysis processing on measured pulse waves to estimate a meal time of a subject. The measuring device 20E transmits the measured pulse waves, the analysis result, the estimated meal time, and the like to the analyzer 10E.

The analyzer 10E includes at least the presenter 13, and presents the pulse waves, the analysis result, a determined message, and the like received from the measuring device 20E. That is, in the analysis system 1E, the analyzer 10E functions as a presentation device that presents pulse waves, analysis results, determined messages, and the like.

Thus, the same effects as those of the above-described analysis system 1 can be exhibited.

In the analysis system 1E, the analyzer 10E may include a part of the functions of the controller 12 (for example, the time estimator 123) and a part of the storage 14 (that is, the log data 142). When this configuration is adopted, the measuring device 20E performs analysis processing on the measured pulse waves and transmits the measured pulse waves and the analysis result to the analyzer 10E. The analyzer 10E presents information such as the meal time based on the analysis result received from the measuring device 20D.

### Fifth Embodiment

Another embodiment of the present disclosure will be described below. For convenience of description, members having the same functions as those of the members described in the above-described embodiment are denoted by the same reference signs, and description thereof is not repeated. In the present embodiment, description of contents overlapping with those of the above-described embodiment will be omitted.

An analyzer 10F according to the present embodiment acquires a pulse wave of a subject continuously measured in a first period after a meal period in which the subject consumes a meal, and detects a pulse wave before the meal of the subject from acquired pulse waves. Then, the analyzer 10F estimates a metabolic index correlated with the stiffness of the blood vessels of the subject based on a change in the detected pulse wave, and presents a message corresponding to the magnitude of change in the estimated metabolic index in the first period to the subject.

As a result, the analyzer 10F estimates a metabolic index correlated with the stiffness of blood vessels caused by the meal for the subject who consumed the meal and presents a message corresponding to the magnitude of change in the estimated metabolic index in the first period to the subject. Accordingly, the analyzer 10F can enable the subject to recognize the dietary composition in association with the reaction of the subject's body after consuming the meal, and prompt the subject to make an effort to continue or improve his or her own dietary composition and dietary habits.

### Analysis System 1

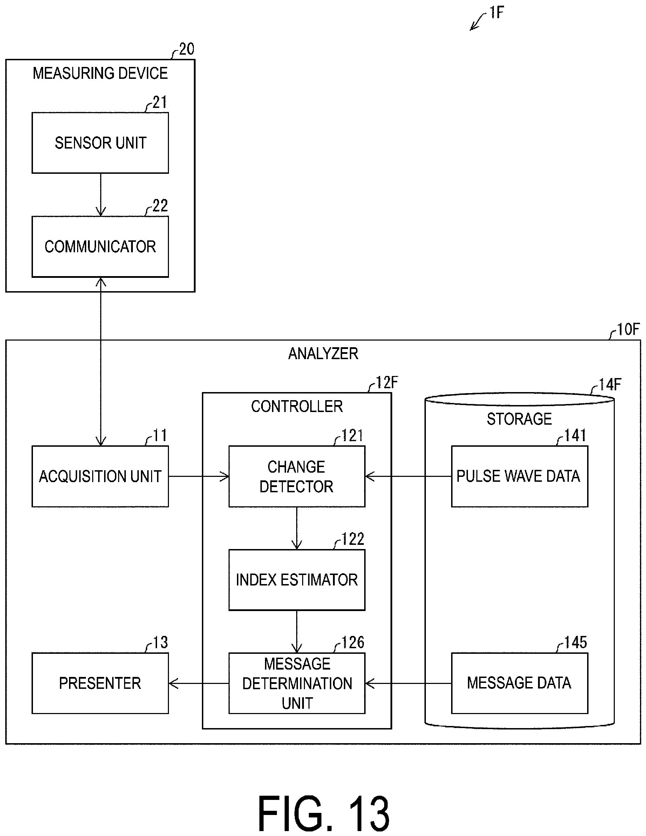

An overview of an analysis system 1F including the analyzer 10 according to the present embodiment will be described with reference to FIG. 13. FIG. 13 is a functional block diagram illustrating a configuration example of the analysis system 1F. As illustrated in FIG. 13, the analysis system 1F includes an analyzer 10F and a measuring device 20.

### Measuring Device 20

The measuring device 20 includes a sensor unit 21 and a communicator 22 as illustrated in FIG. 13.

The sensor unit 21 acquires pulse waves of a subject. More specifically, the sensor unit 21 acquires pulse waves of a subject by detecting a volume change (expansion or contraction) of a blood vessel based on reflected light of light radiated toward the blood vessel of the subject. As the light radiated toward the blood vessel of the subject, for example, light having the infrared wavelength can be used.

The sensor unit 21 may measure pulse waves of the subject at a higher measurement frequency in the first period than in other periods in accordance with an instruction from the analyzer 10F. The measurement frequency may indicate, for example, the number of measurement operations in a predetermined period.

### Analyzer 10F

The analyzer 10F will be described with reference to FIG. 13. As illustrated in FIG. 13, the analyzer 10F includes an acquisition unit 11, a controller 12F, a presenter 13, and a storage 14F. The controller 12F includes a change detector (detector) 121, an index estimator 122, and a message determination unit 126.

The acquisition unit 11 acquires pulse wave data of the subject continuously measured by the measuring device 20 from the measuring device 20. A first period may be set by a user from predetermined options or may be arbitrarily set. Alternatively, the first period may be automatically set based on pulse wave data of the past. Alternatively, when the user does not select the first period, the first period may be automatically set from pulse wave data of the past.

The controller 12F controls the analyzer 10F, and includes the change detector 121, the index estimator 122, and the message determination unit 126 as described above.

The message determination unit 126 determines a message corresponding to the magnitude of change in the metabolic index estimated by the index estimator 122 in the first period.

For example, if a change in the metabolic index in the first period exceeds a negative evaluation criterion (fifth criterion), the message determination unit 126 may determine a message indicating a negative evaluation on the dietary composition consumed by the subject as a message corresponding to the magnitude of change. The message may be verbal language, numerals, or a combination thereof. The determined message may be, for example, "a little more", "unhappy", or "too bad" in the case of verbal language, or may be "20 points" or "20/100 points" in the case of numerals. The message may be something that allows the subject to specifically review his or her dietary habits, such as a message saying "Your score has greatly decreased. What did you eat for your meal?".

The negative evaluation criterion may be, for example, a case when a post-meal ratio to a pre-meal ratio is 1/2 or less. Here, the pre-meal ratio and the post-meal ratio will be described with reference to FIG. 4. As described above, the graph 401 in FIG. 4 is an example of the pulse wave before a meal, and the graph 402 is an example of the pulse wave in the first period. The pre-meal ratio is the ratio Y1/X1 of the second peak Y1 to the first peak X1 of the graph 401. The post-meal ratio is the ratio Y2/X2 of the second peak Y2 to the first peak X2 in the graph 402.

Returning to FIG. 13, when the change in the metabolic index in the first period does not exceed a positive evaluation criterion (sixth criterion), the message determination unit 126 may determine a message indicating a positive evaluation on the dietary composition consumed by the subject as a message corresponding to the magnitude of change. The message may be verbal language, numerals, or a combination thereof. The determined message may be, for example, "great", "favorable", or "good" in the case of verbal language, or may be "85 points" or "85/100 points" in the case of numerals.

Examples of positive evaluation criteria include, for example, a post-meal ratio to a pre-meal ratio exceeding 1/2.

The message determination unit 126 may determine a message including information related to the mode of change in the metabolic index as a message corresponding to the magnitude of change. The mode of change in the metabolic index is, for example, a rising manner, a slope, a peak height, an absolute amount, or the like. An example of a message including information about the mode of change in the metabolic index includes "The shape of the pulse wave has changed sharply. Has the dietary composition consumed been appropriate?".

The message determination unit 126 may determine a message including an index indicating the state of the subject other than the metabolic index as a message corresponding to the magnitude of change. The state of the subject is, for example, a physical condition of the subject.

For example, the message determination unit 126 may use the degree of exercise or the like of the subject acquired from the acceleration sensor as the state of the subject, and determine a message indicating "It seems that you did not exercise much today. Please adjust both your diet and exercise".

The message determination unit 126 may determine a message indicating "You seem to be very tired today" as the state of the subject from the heart rate and the body temperature of the subject acquired from the body temperature sensor.

The message determination unit 126 may determine a message such as "It seems that the time from the meal to the start of sleep was short" from the detection result of the acceleration sensor or the like.

For example, the analyzer 10F specifies that the body of the subject has not been fully recovered based on the fact that the heart rate of the subject when resting at the previous night was high based on the heard rate of the subject acquired on the previous day and the detection result of the acceleration sensor or the like, that the sleep time was short, or that the estimated sitting time in one day was long (the subject was sitting for a long time showing a low degree of activity), or the like.

When the analyzer 10F specifies that the body of the subject has not been fully recovered, the analyzer 10F determines and presents a message as described above.

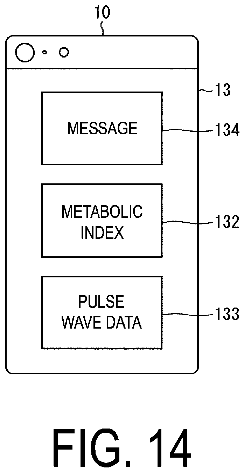

The presenter 13 presents the message determined by the message determination unit 126 to the subject. FIG. 14 is a diagram illustrating a presentation example presented by the analyzer 10F. In the presentation example illustrated in FIG. 14, a message is presented in an area 134 of the presenter 13, a metabolic index is presented in an area 132, and pulse wave data is presented in an area 133.

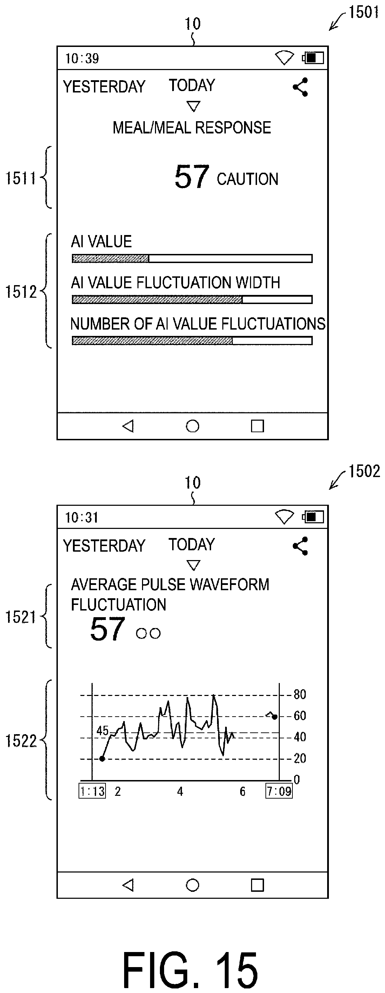

FIG. 15 is a diagram illustrating another presentation example presented by the analyzer 10F. The analyzer 10F may give presentation as illustrated in FIG. 15. Reference numeral 1501 in FIG. 15 denotes a presentation example including an evaluation for a meal. In 1501 of FIG. 15, an evaluation on a meal is presented in an area 1511, and information on an AI value as a metabolic index is presented in an area 1512. Reference numeral 1502 in FIG.15 denotes a presentation example of a variation of a metabolic index. An AI value as a metabolic index is presented in an area 1521 in reference numeral 1502 in FIG. 15, and a varying state of the AI value is presented in an area 1522.

The storage 14 stores data used by the analyzer 10F, and includes pulse wave data 141 and message data 145.

The message data 145 is data used by the message determination unit 126 to determine a message.

As described above, the analyzer 10F according to the present disclosure includes the acquisition unit 11 that acquires a pulse wave of the subject continuously measured by using a wearable device in the first period after the meal period in which the subject consumed a meal. The analyzer 10F also includes the change detector 121 that detects the pulse wave of the subject before a meal from pulse waves acquired by the acquisition unit 11. The analyzer 10F includes the index estimator 122 that estimates a metabolic index correlated with the stiffness of the blood vessel of the subject based on the change in the pulse wave detected by the change detector 121. The analyzer 10F includes the message determination unit 126 that determines a message corresponding to the magnitude of change in the metabolic index estimated by the index estimator 122 in the first period. The analyzer 10F includes the presenter 13 that presents the message determined by the message determination unit 126 to the subject.

As a result, the subject can be made to recognize the dietary composition in association with the reaction of the subject's body after consuming the meal. The subject can be made to recognize a problem of his/her dietary composition and to establish proper dietary habits.

### Processing Flow

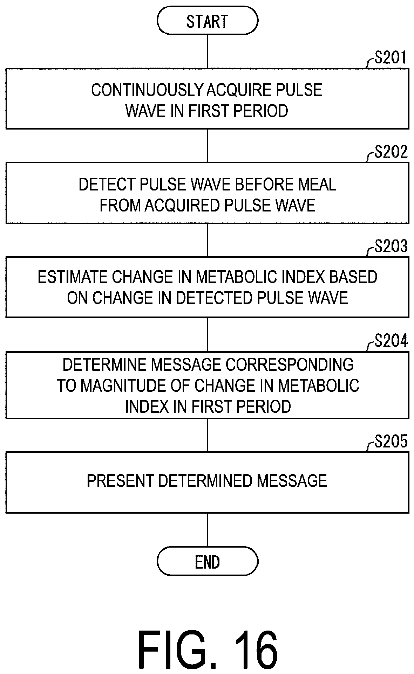

The flow of processing of the analyzer 10F will be described with reference to FIG. 16. FIG. 16 is a flowchart showing an example of the flow of processing by the analyzer 10F. Since the processing from S201 to S203 shown in FIG. 16 is the same as the processing from S101 to S101 shown in FIG. 7, description thereof will be omitted. After performing the processing of S203, the message determination unit 126 determines a message corresponding to the magnitude of change in the metabolic index estimated by the index estimator 122 in the first period (S204; message determination step). Then, the presenter 13 presents the message determined by the message determination unit 126 (S205; presentation step).

The above is the flow of the processing by the analyzer 10F.

### Sixth Embodiment

Another embodiment of the present disclosure will be described below. For convenience of description, members having the same functions as those of the members described in the above-described embodiment are denoted by the same reference signs, and description thereof is not repeated.

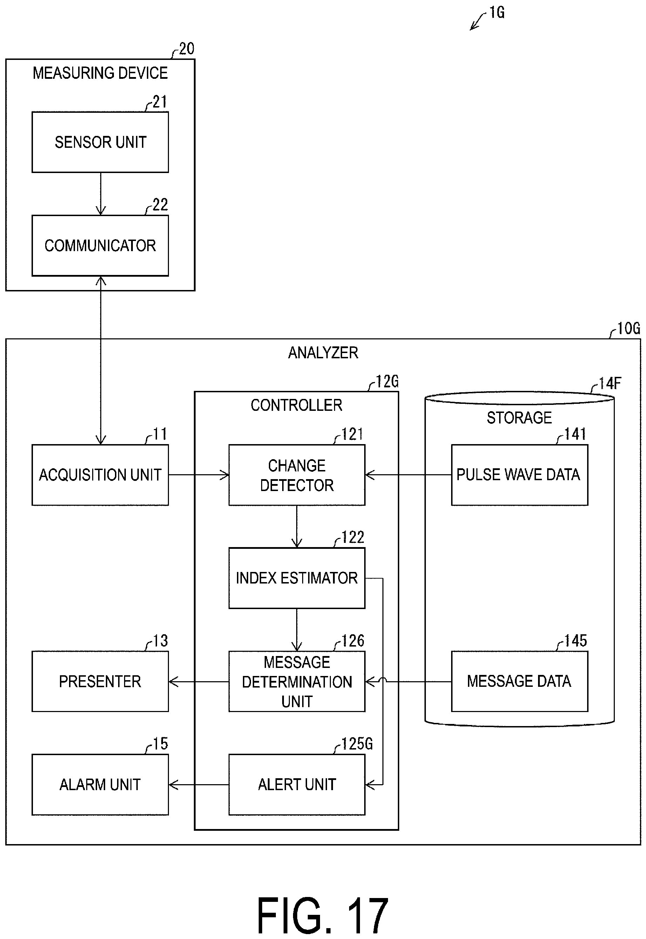

FIG. 17 is a functional block diagram illustrating a principal configuration example of an analysis system 1G according to the present embodiment. The analysis system 1G includes a measuring device 20 and an analyzer 10G. As illustrated in FIG. 17, the analyzer 10G is different from the analyzer 10F in the fifth embodiment described above in that a controller 12G includes an alert unit 125G and the analyzer 10G includes an alarm unit 15.

When the metabolic index estimated by the index estimator 122 exceeds an alert criterion (seventh criterion), the alert unit 125G outputs an alert to the subject from the alarm unit 15. For example, when the metabolic index is a value related to the dietary composition, the seventh criterion may be a criterion by which the dietary composition of the subject adversely affects his or her health when the metabolic index exceeds the criterion.

### Seventh Embodiment

Another embodiment of the present disclosure will be described below. For convenience of description, members having the same functions as those of the members described in the above-described embodiment are denoted by the same reference signs, and description thereof is not repeated.

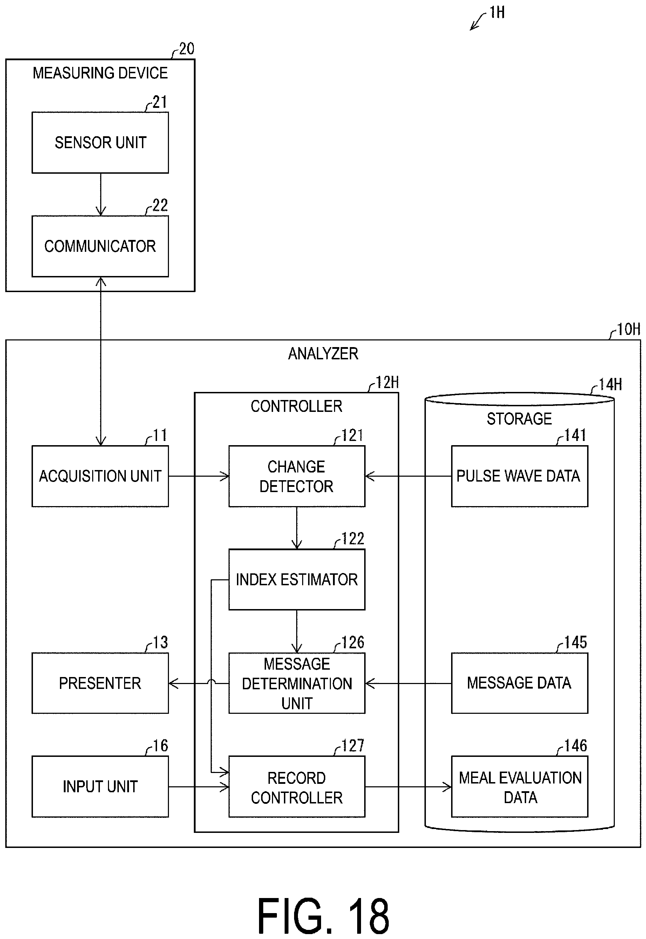

FIG. 18 is a functional block diagram illustrating a configuration example of an analysis system 1H according to the present embodiment. The analysis system 1H includes a measuring device 20 and an analyzer 10H. As illustrated in FIG. 18, the analyzer 10H is different from the analyzer 10F in the above-described fifth embodiment in that a controller 12H includes a record controller 127, a storage 14H stores meal evaluation data 146, and the analyzer 10H includes an input unit 16.

The input unit 16 receives, from a subject, input of the dietary composition consumed by the subject. The input of the dietary composition may be performed by inputting characters, or may be selected from options prepared in advance. The dietary composition previously input may be selected. The dietary composition may be input together with the time of the meal consumed. The input dietary composition may be items such as "rice, vegetable, and miso soup" or the like, or may be specific names such as "white rice, lettuce, tomato, carrot, wakame seaweed, and tofu". The order of consumption may be recorded.

The input unit 16 may receive the input of the dietary composition of the subject at a predetermined time after the meal or may receive the input before the meal.

The record controller 127 stores the input dietary composition in association with the metabolic index estimated by the index estimator 122 as the meal evaluation data 146 in the storage 14H.

The message determination unit 126 may determine, as a message to be presented to the subject, a message with content including an evaluation on the meal of the subject based on a change in the metabolic index in a first period. The message with a content including an evaluation on the meal of the subject is, for example, "The meal time may have been too short. Consuming a meal slowly is ideal for achieving a mild change in sugar metabolism" by using the length of the meal time estimated from the detection result of the acceleration sensor.

The message determination unit 126 may determine, as a message to be presented to the subject, a message including the evaluation result of behavior related to a meal of the subject and advice with a content according to the evaluation result. For example, the message determination unit 126 may determine a message indicating "It seems that there was a lot of sugar. Please control the amount of sugar in one meal. It is important to start with vegetables" according to the dietary composition input by the subject. A relationship between the dietary composition and nutrients may be stored in advance.

### Third Variation

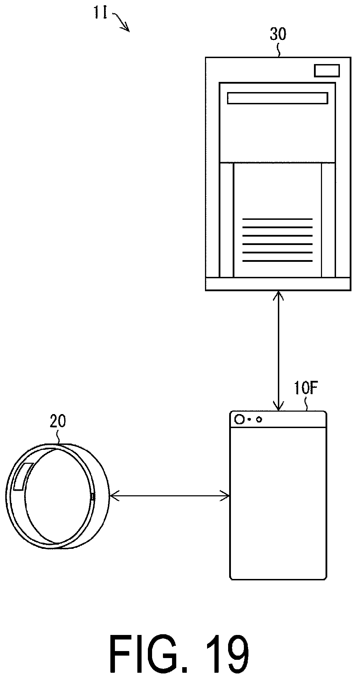

FIG. 19 is a diagram illustrating an example of an overview of an analysis system 1I including a server 30. As illustrated in FIG. 19, the analysis system 1I further includes the server 30 in addition to the analyzer 10F and the measuring device 20 described above.

The analyzer 10F and the server 30 are communicatively connected to a network.

The server 30 performs a part or all of the analysis processing performed by the analyzer 10F. Since the server 30 performs the analysis processing, the analyzer 10F can reduce processing load.

### Fourth Variation

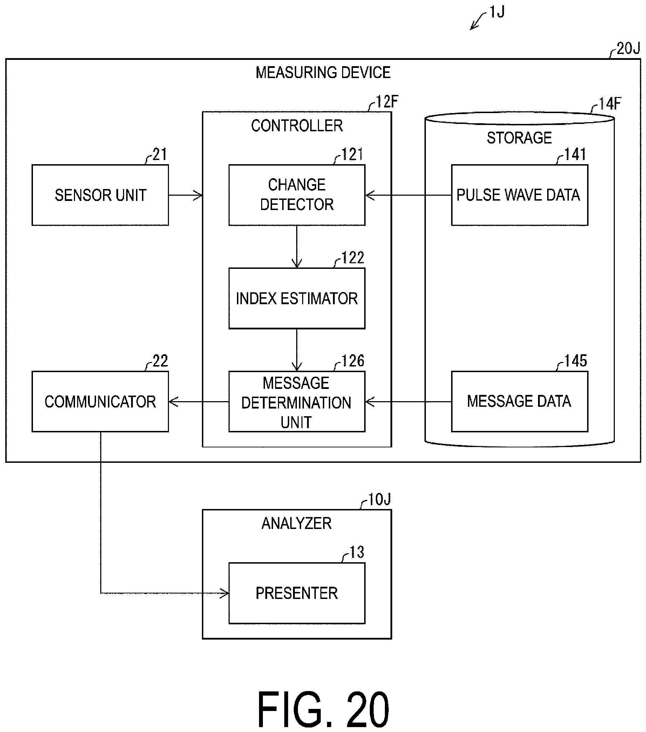

The analyzer 10F analyzes pulse waves measured by the measuring device 20 in the embodiments described above. However, the present disclosure is not limited thereto, and a part of the functions of the analyzer 10F may be assigned to the measuring device 20. An analysis system 1J employing such a configuration will be described with reference to FIG. 20. FIG. 20 is a functional block diagram illustrating a configuration example of an analysis system 1J. A measuring device 20J of the analysis system 1J measures and analyzes pulse waves, and an analyzer 10J acquires the analysis result acquired from the measuring device 20J and presents the analysis result on the presenter 13.

As illustrated in FIG. 20, the measuring device 20J includes a controller 12F (a change detector 121, an index estimator 122, and a message determination unit 126) and a storage 14F (pulse wave data 141 and message data 145) in addition to a sensor unit 21 and a communicator 22. The measuring device 20J performs analysis processing on the measured pulse waves and determines a message to be presented to the analyzer 10J based on the analysis result. The measuring device 20J transmits the measured pulse waves, the analysis result, the determined message, and the like to the analyzer 10J.

The analyzer 10J includes at least the presenter 13, and presents the pulse waves, the analysis result, a determined message, and the like received from the measuring device 20J. That is, in the analysis system 1J, the analyzer 10J functions as a presentation device that presents pulse waves, analysis results, determined messages, and the like.

Thus, the same effects as those of the above-described analysis system 1F can be exhibited.

In the analysis system 1J, the analyzer 10J may include some of the functions of the controller 12F (for example, the message determination unit 126) and a part of the storage 14F (that is, the message data 145). When this configuration is adopted, the measuring device 20J performs analysis processing on the measured pulse waves and transmits the measured pulse waves and the analysis result to the analyzer 10J. The analyzer 10J determines a message to be presented based on the analysis result received from the measuring device 20J and presents the message.

### Implementation Example by Software

Functions of the analyzers 10 and 10A to 10J and the measuring devices 20E and 20J (hereinafter, referred to as "devices") can be implemented by a program for causing a computer to function as the devices and for causing the computer to function as each control block (particularly, each unit included in the controllers 12, 12A, 12B, 12C, 12F, 12G, and 12H) of the devices.

In this case, the devices include a computer including at least one control device (for example, a processor) and at least one storage device (for example, a memory) as hardware for executing the program. As the control device and the storage device execute the program, the functions described in the embodiments described above are implemented.

The program may be recorded on one or a plurality of computer-readable non-transitory recording media. This recording media may be or need not be included in the devices. In the latter case, the program may be supplied to the devices via any wired or wireless transmission medium.

Some or all of the functions of the control blocks can be implemented by logic circuits. For example, an integrated circuit in which logic circuits functioning as the control blocks are formed is also included in the scope of the present disclosure. In addition to this, for example, a quantum computer can implement the functions of the control blocks.

Each of the processing operations described in the above-described embodiments may be executed by artificial intelligence (AI). In this case, the AI may operate in the control devices, or may operate in another device (for example, an edge computer or a cloud server).

In the present disclosure, the invention has been described above based on the various drawings and examples. However, the invention according to the present disclosure is not limited to each embodiment described above. That is, the embodiments of the invention according to the present disclosure can be modified in various ways within the scope illustrated in the present disclosure, and embodiments obtained by appropriately combining the technical means disclosed in different embodiments are also included in the technical scope of the invention according to the present disclosure. In other words, a person skilled in the art can easily make various variations or modifications based on the present disclosure. Note that these variations or modifications are included within the scope of the present disclosure.

### Conclusion

The analyzers 10, 10A, 10B, 10C, 10D, 10F, 10G, 10H, and 10I, and the measuring devices 20E and 20J according to a first aspect of the present disclosure include the acquisition unit 11 that acquires a pulse wave of a subject continuously measured by a wearable device (the measuring devices 20, 20A, 20E, and 20J) in a first period after a meal period in which the subject consumes a meal, the change detector 121 (detector) that detects a pulse wave before the meal of the subject from pulse waves acquired by the acquisition unit 11, and the index estimator 122 that estimates a change in a metabolic index correlated with stiffness of a blood vessel of the subject based on change in the pulse wave detected by the change detector 121.

The analyzers 10, 10A, 10B, 10C, and 10D, and the measuring device 20E according to a second aspect of the present disclosure may include the time estimator 123 that estimates a meal time that is an arbitrary time in the meal period based on a change in the metabolic index in the first aspect.

In the analyzers 10, 10A, 10B, 10C, and 10D, and the measuring device 20E according to a third aspect of the present disclosure, the time estimator 123 may estimate at least one of a meal start time and a meal end time of the subject based on a combination of the metabolic index and another index in the second aspect.

In the analyzers 10, 10A, 10B, 10C, and 10D, and the measuring device 20E according to a fourth aspect of the present disclosure, the other index may be at least one of an acceleration of a body motion of the subject and a body surface temperature of the subject in the third aspect.

In the analyzers 10, 10A, 10B, 10C, and 10D, and the measuring device 20E according to a fifth aspect of the present disclosure, the time estimator 123 may estimate the meal time based on a combination of the metabolic index and a training result based on a past behavior record of the subject in any one of the second to fourth aspects.

In the analyzers 10, 10A, 10B, 10C, and 10D, and the measuring device 20E according to a sixth aspect of the present disclosure, the time estimator 123 may estimate the meal time of the subject based on a relationship between a past meal time of the subject and information indicating a state of the subject at the past meal time of the subject in addition to the change in the metabolic index in any one of the second to fifth aspects.

In the analyzers 10, 10A, 10B, 10C, and 10D, and the measuring device 20E according to a seventh aspect of the present disclosure, the time estimator 123 may estimate the meal time based on the change in the metabolic index as a first criterion, the metabolic index being estimated based on a pulse wave of the subject measured in the past at the time of wake-up or fasting in any one of the second to sixth aspects.

The analyzers 10, 10A, 10B, 10C, and 10D, and the measuring device 20E according to an eighth aspect of the present disclosure may further include the alert unit 125 that outputs a predetermined alert to the subject when the metabolic index estimated by the index estimator 122 falls below a second criterion in any one of the second to seventh aspects.

The analyzers 10, 10A, 10B, 10C, and 10D, and the measuring device 20E according to a ninth aspect of the present disclosure may further include the alert unit 125 that outputs an alert prompting the subject to consume food when the time that has elapsed from the meal time estimated by the time estimator 123 exceeds a third criterion in any one of the second to eighth aspects.

The analyzers 10, 10A, 10B, 10C, and 10D, and the measuring device 20E according to a tenth aspect of the present disclosure may further include the alert unit 125 that outputs advice on the next meal to the subject when the metabolic index estimated by the index estimator 122 falls below a fourth criterion in any one of the second to ninth aspects.

The analyzers 10F, 10G, 10H, and 10I, and the measuring device 20J according to an 11th aspect of the present disclosure include the message determination unit 126 that determines a message corresponding to the magnitude of change in a metabolic index estimated by the index estimator 122 in the first period, and the presenter 13 that presents the message determined by the message determination unit 126 to the subject in the first aspect.

In the analyzers 10F, 10G, 10H, and 10I, and the measuring device 20J according to a 12th aspect of the present disclosure, the message determined by the message determination unit 126 may be a message indicating a negative evaluation on the dietary composition consumed by the subject when the change in the metabolic index in the first period exceeds a fifth criterion in the 11th aspect.

In the analyzers 10F, 10G, 10H, and 10I, and the measuring device 20J according to a 13th aspect of the present disclosure, the message determined by the message determination unit 126 may be a message indicating a positive evaluation on the dietary composition consumed by the subject when the change in the metabolic index in the first period does not exceed a sixth criterion in the 11th aspect or 12th aspect.

In the analyzers 10F, 10G, 10H, and 10I, and the measuring device 20J according to a 14th aspect of the present disclosure, pulse waves of the subject acquired by the acquisition unit 11 may include the meal period of the subject in any one of the 11th to 13th aspects.

In the analyzers 10F, 10G, 10H, and 10I, and the measuring device 20J according to a 15th aspect of the present disclosure, pulse waves of the subject acquired by the acquisition unit 11 may include a second period before the meal period in any one of the 11th to 14th aspects.

In the analyzers 10F, 10G, 10H, and 10I, and the measuring device 20J according to a 16th aspect of the present disclosure, the message determined by the message determination unit 126 may include information about a mode of change in the metabolic index in any one of the 11th to 15th aspects.

In the analyzers 10F, 10G, 10H, and 10I, and the measuring device 20J according to a 17th aspect of the present disclosure, the message determined by the message determination unit 126 may include an index indicating a state of the subject other than the metabolic index in any one of the 11th to 16th aspects.

The analyzer 10G according to an 18th aspect of the present disclosure may include the alert unit 124 that outputs an alert to the subject when the metabolic index estimated by the index estimator 122 exceeds a seventh criterion in any one of the 11th to 17th aspects.

In the analyzer 10H according to a 19th aspect of the present disclosure, the message determined by the message determination unit 126 may include an evaluation on the meal based on the change in the metabolic index in the first period in any one of the 11th to 18th aspects.

The analyzer 10H according to a 20th aspect of the present disclosure may further include the record controller 127 that records the content of the meal in association with the metabolic index estimated based on the pulse waves in the first period in any one of the 11th to 19th aspects.

The analyzer 10H according to a 21st aspect of the present disclosure may include the input unit 16 that receives an input of the content of the meal from the subject in any one of the 11th to 20th aspects.

In the analyzer 10H according to a 22nd aspect of the present disclosure, the message determined by the message determination unit 126 may include an evaluation result of behavior related to a meal of the subject and advice indicating a content corresponding to the evaluation result in any one of the 11th to 21st aspects.

An analysis method according to a 23rd aspect of the present disclosure includes an acquisition step of acquiring a pulse wave of a subject continuously measured by a wearable device (measuring devices 20, 20A, 20E, and 20J) in a first period after a meal period in which the subject consumes a meal, a detection step of detecting a pulse wave of the subject before a meal from acquired pulse waves, and an index estimation step of estimating a change in a metabolic index correlated with stiffness of a blood vessel of the subject based on change in the detected pulse wave.

The analysis method according to a 24th aspect of the present disclosure includes a time estimation step of estimating a meal time which is an arbitrary time in the meal period based on the change in the metabolic index in the 23rd aspect.

The analysis method according to a 25th aspect of the present disclosure includes a message determination step of determining a message corresponding to a magnitude of change in the metabolic index estimated in the index estimation step in the first period, and a presentation step of presenting the message determined in the message determination step to the subject in the 23rd aspect.

### REFERENCE SIGNS

1, 1A to 1J Analysis system
10, 10A to 10J Analyzer
11 Acquisition unit
12 Controller
121 Change detector (detector)
122 Index estimator
123, 123A, 123B Time estimator
124 Acceleration detector
125, 125G Alert unit
126 Message determination unit
127 Record controller
13 Presenter
14 Storage
141 Pulse wave data
142 Log data
143 Behavior record data
144 Estimation model
145 Message data
146 Meal evaluation data
15 Alarm unit
16 Input unit
20, 20A, 20E, 20J Measuring device
21 Sensor unit
22 Communicator
23 Acceleration sensor
30 Server

## Claims

1. An analyzer comprising:
an acquisition unit configured to acquire a pulse wave of a subject continuously measured by a wearable device in a first period after a meal period in which the subject consumes a meal;
a detector configured to detect a pulse wave before the meal of the subject from pulse waves acquired by the acquisition unit; and
an index estimator configured to estimate a change in a metabolic index correlated with stiffness of a blood vessel of the subject based on a change in the pulse wave detected by the detector.

2. The analyzer according to claim 1, further comprising:
a time estimator configured to estimate a meal time which is an arbitrary time in the meal period based on the change in the metabolic index.

3. The analyzer according to claim 2, wherein
the time estimator estimates at least one of a meal start time and a meal end time of the subject based on a combination of the metabolic index and another index.

4. The analyzer according to claim 3, wherein
the other index is at least one of an acceleration of a body motion of the subject and a body surface temperature of the subject.

5. The analyzer according to any one of claims 2 to 4, wherein
the time estimator estimates the meal time based on a combination of the metabolic index and a training result based on a past behavior record of the subject.

6. The analyzer according to any one of claims 2 to 5, wherein
the time estimator estimates the meal time of the subject based on a relationship between a past meal time of the subject and information indicating a state of the subject at the past meal time in addition to the change in the metabolic index.

7. The analyzer according to any one of claims 2 to 6, wherein
the time estimator estimates the meal time based on the change in the metabolic index as a first criterion, the metabolic index being estimated based on a pulse wave of the subject measured in the past at the time of wake-up or fasting.

8. The analyzer according to any one of claims 2 to 7, further comprising:
an alert unit configured to output a predetermined alert to the subject when the metabolic index estimated by the index estimator falls below a second criterion.

9. The analyzer according to any one of claims 2 to 8, further comprising:
an alert unit configured to output an alert prompting the subject to consume food when a time that has elapsed from the meal time estimated by the time estimator exceeds a third criterion.

10. The analyzer according to any one of claims 2 to 9, further comprising:
an alert unit configured to output advice on the next meal to the subject when the metabolic index estimated by the index estimator falls below a fourth criterion.

11. The analyzer according to claim 1, further comprising:
a message determination unit configured to determine a message corresponding to a magnitude of change in a metabolic index estimated by the index estimator in the first period; and
a presenter configured to present the message determined by the message determination unit to the subject.

12. The analyzer according to claim 11, wherein
the message determined by the message determination unit is a message indicating a negative evaluation of dietary composition consumed by the subject when the change in the metabolic index in the first period exceeds a fifth criterion.

13. The analyzer according to claim 11 or 12, wherein
the message determined by the message determination unit is a message indicating a positive evaluation of the dietary composition consumed by the subject when the change in the metabolic index in the first period does not exceed a sixth criterion.

14. The analyzer according to any one of claims 11 to 13, wherein
a pulse wave of the subject acquired by the acquisition unit comprises the meal period of the subject.

15. The analyzer according to any one of claims 11 to 14, wherein
a pulse wave of the subject acquired by the acquisition unit comprises a second period before the meal period.

16. The analyzer according to any one of claims 11 to 15, wherein
the message determined by the message determination unit comprises information related to a mode of change in the metabolic index.

17. The analyzer according to any one of claims 11 to 16, wherein
the message determined by the message determination unit comprises an index indicating a state of the subject other than the metabolic index.

18. The analyzer according to any one of claims 11 to 17, further comprising: an alert unit configured to output an alert to the subject when the metabolic index estimated by the index estimator exceeds a seventh criterion.

19. The analyzer according to any one of claims 11 to 18, wherein
the message determined by the message determination unit comprises an evaluation on the meal based on the change in the metabolic index in the first period.

20. The analyzer according to any one of claims 11 to 19, further comprising:
a record controller configured to record content of the meal in association with the metabolic index estimated based on the pulse wave in the first period.

21. The analyzer according to any one of claims 11 to 20, comprising:
an input unit configured to receive an input of content of the meal from the subject.

22. The analyzer according to any one of claims 11 to 21, wherein
the message determined by the message determination unit comprises an evaluation result on behavior related to a meal of the subject and advice with content based on the evaluation result.

23. An analysis method comprising:
acquiring a pulse wave of a subject continuously measured by a wearable device in a first period after a meal period in which the subject consumes a meal;
detecting a pulse wave of the subject before the meal from the acquired pulse wave; and
estimating a change in a metabolic index correlated with stiffness of a blood vessel of the subject based on a change in the detected pulse wave.

24. The analysis method according to claim 23, comprising:
estimating a meal time which is an arbitrary time in the meal period based on a change in the metabolic index.

25. The analysis method according to claim 23, comprising:
determining a message corresponding to a magnitude of change in the metabolic index estimated in the index estimating in the first period; and
presenting the message determined in the message determining to the subject.
